# EUROPEAN PATENT APPLICATION

(11) **EP 1 232 756 A1**
(43) Date of publication of application: **21.08.2002**
(21) Application number: 00969983.6
(22) Date of filing: 23.10.2000
(51) Int. Cl.: A61K 45/00, A61K 31/352, A61P 43/00, A61P 5/00, C07D 311/62

(54) **DRUGS, DRINKS OR FOODS**

(30) Priority: 29.10.1999 JP 30801399; 25.07.2000 JP 2000224469
(71) Applicant: Takara Shuzo Co, Ltd., Kyoto-shi, Kyoto 612-8061 (JP)
(72) Inventor: NISHIYAMA, Eiji, Moriyama-shi, Shiga 524-0102 (JP); OHNOGI, Hiromu, Terado-cho, Muko-shi, Kyoto 617-0002 (JP); KONDO, Akihiro, Kusatsu-shi, Shiga 525-0025 (JP); OYASHIKI, Haruo, Otsu-shi, Shiga 520-2276 (JP); SAGAWA, Hiroaki, Kusatsu-shi, Shiga 525-0025 (JP); MIZUTANI, Shigetoshi, Gamo-gun, Shiga 521-1322 (JP); KATO, Ikunoshin, Uji-shi, Kyoto 611-0028 (JP)
(74) Representative: Vossius, Volker, Dr.
(86) International application number: JP0007372
(87) International publication number: WO01032212

(57) **Abstract**

Remedies or preventives for diseases caused by the binding of agonists (endocrine disruptors, etc.) to the estrogen receptor, characterized by containing, as the active ingredient, antagonists such as catechins (for example, epigallocatechin gallate) having the antagonism to the estrogen receptor agonists.

## Description

### Technical Field

The present invention relates to pharmaceutical compositions, drinks or foods for treating or preventing diseases caused through estrogen receptor.

### Background Art

Estrogen is an endogenous hormone which has a wide variety of important functions in human physiology. This hormone mainly influences the reproduction systems in both male and female individuals. For example, estrogen is indispensable for the processes of pregnancy and lactation in adult female individuals. It reduces the risks of heart stroke and osteoporosis. Furthermore, the ratio of estrogen to androgen before and early after birth regulates sexual differentiation and appropriate formation of proper genital organs. Estrogen also has connection with proliferation (i.e., cancer) of mammary and uterine cells. Estrogen-dependent breast cancer is one known disease state in this regard. Other known estrogen-dependent disease states include metrofibroma, endometritis, and proliferation of smooth muscle cells. Estrogen regulates sperm production in male individuals. Abnormality of estrogen results in decrease in sperm number per unit volume (oligospermia). Since androgen is known to have connection with prostatomegaly, treatment of prostatomegaly by administration of an antiandrogenic agent has been examined. Recently, existence of estrogen receptor on some of prostatic cells was confirmed. Then, use of mepartricin, a substance that suppresses the expression of estrogen receptor, as an agent for suppressing prostatomegaly has been examined (The Prostate, 38:17-27, 1999). However, no substance that competes with estrogen for binding to estrogen receptor has been reported to suppress prostatomegaly.

Recent studies have shown that estrogen has many homeostatic functions in addition to those connected with functions of genital or sexual tissues in female individuals. In fact, it is known that estrogen receptor in male individuals has a receptor site and a DNA recognition site, and has abilities to form many tissues participating in estrogen and the cardiovascular system. However, the exactly essential functions of estrogen in male and female individuals other than those in the aspects of formation are currently almost unknown because such studies have been just begun.

Biologically active estrogen exerts its properties upon binding to the intracellular receptor. The receptor and the ligand bound thereto are transferred into the nucleus in the cell. The complex binds to the DNA at a specific recognition site to allow specific gene(s) to express, thereby exhibiting the activities. However, almost nothing is known about the binding to the receptor and the regulation.

On the other hand, hormone-like activities or antihormonal activities have been recently observed for some chemical substances released into the environment. Such substances are collectively called endocrine disruptors (or environmental hormones). For example, chlorine pesticides, health products for animals, plastics, by-products generated upon incineration, antimicrobial agents, herbicides, industrial chemical substances such as bisphenol-A and nonylphenol, and the like are known to influence organisms. Altered reproductive potential (in particular, conversion of male into female), decreased reproductive potential, decreased hatchability, decreased survival rate of offspring, abnormal reproductive behavior and the like have been reported to be resulted from the influences of such substances on the ecosystem of wild animals. Decrease in sperm number, endometriosis, infertility, ovarian cancer, uterine cancer, prostatic cancer and the like have been suspected to be resulted from the influences of the substances on the human health.

It has been reported that some of the endocrine disruptors in the environment bind to the receptor for estrogen (a sexual hormone) to exhibit estrogenic activities (or to act as an agonist). For example, with respect to the reduced reproductive potential including reduced quality of human semen, it was reported that sperm number had been reduced almost by half during the past 50 years (BMJ, 305: 609-613, 1992).

The uterotrophic assay using juvenile rats is one of general animal tests for determining the endocrine-disrupting activities (or so-called environmental-hormonal activities) of suspected substances. This assay utilizes the fact that growth of a uterus is controlled by estrogen. Specifically, the blood concentration of endogenous estrogen, 17β-estradiol, is kept low in prepubertal juvenile animals, and a uterus is enlarged in response to exogenous estrogen in a dose-dependent manner (T. Inoue (ed.), "Naibunpitsu kakuran kagaku busshitsu no seibutsu shiken kenkyuuhou" (Biological assays for endocrine-disrupting chemical substances) (2000) Springer-Verlag Tokyo). Accordingly, this assay is conducted during a limited period from 18 days after birth, when rats can be weaned, to 25 days after birth, i.e., prior to puberty. Nevertheless, OECD recommends this assay for the following reasons: the period is short; no special technique is required; and reproducible results are obtained (OECD VALIDATION WORK ON IN-VIVO UTEROTROPHIC SCREENING ASSAY, Protocol for demonstration of dose-response of the uterotrophic assay using the reference oestrogen ethinyl oestradiol (1999)). Using this assay, administration of bisphenol-A or 4-nonylphenol, which had been suspected to have endocrine-disrupting activities, to juvenile rats was reported to increase uterine weight (Environ Health Perspect, 106:719-720, 1998; Toxicological Sciences, 54:154-167, 2000).

On the other hand, it has been considered that use of a substance that binds to estrogen receptor in a manner antagonistic against the substances that bind to estrogen receptor and exhibit endocrine-disrupting activities would be effective in avoiding the influences of the endocrine disruptors. For example, isoflavone from soybeans is known to bind to estrogen receptor so strongly as endocrine disruptors do. When isoflavone is orally taken, it competes with endocrine disruptors in the living body, and exhibits estrogenic activities, resulting in influences similar to those of the endocrine disruptors (Environ Health Perspect, 106:369-373, 1998).

It is described that fatty acids, catechins, gallic acid derivatives and the like inhibit the activity of 5α-reductase, and are effective for the treatment of prostatic cancer and prostatomegaly in a patent and patent applications by Shutsung Liao et al. (Arch Development Corp., USA; US 5605929, WO 99/22728, JP-A 11-507022). None of these publications contain disclosure of treatment experiments including a study using an animal model, and only presume the effects based on prior art literatures (e.g., P. K. Siiteri et al. (1970) J. Clin. Invest., 49:1737-1745, P. C. Walsh et al. (1983) J. Clin. Invest., 72:1772-1777, E. Stoner et al. (1992) J. Urol. 147:1298-1302, G. J. Gormley et al. (1992) N. Eng. J. Med., 327:1185-1191, N. Bruchovsky et al. (1988) J. Clin. Endocrinol. Metab., 67:806-816).

The distributions of two known isozymes of 5α-reductase are quite different among animal species or tissues (A. E. Thigpen et al. (1993) J. Clin. Invest., 92:903-910; David W. Russell & Jean D. Wilson (1994) Annu. Rev. Biochem., 63:25-61). The type 2 5α-reductase has connection with prostatic cancer and prostatomegaly in humans, whereas Liao et al. observed the inhibition of the type 1 5α-reductase by catechins or gallic acid derivatives. Thus, the effectiveness of catechins or gallic acid derivatives on prostatic cancer or prostatomegaly in humans is not shown based on the data of Liao et al. Furthermore, it is described in JP-A 11-507022 that the gallate groups in gallic acid derivatives or catechins do not have connection with the inhibition of 5α-reductase and that structures of portions in catechins other than the gallate groups are important.

In addition, Liao et al. reported, with respect to the activities of inhibiting 5α-reductase of gallic acid derivatives or catechins, that structures of portions in catechins other than the gallate groups are important for the activities, and gallic acid derivatives do not have an activity of inhibiting 5α-reductase (US 5605929). Liao et al. also reported that the 5α-reductase activity is not inhibited under whole cell assay conditions (WO 99/22728).

As described above, to date, catechins or gallic acid derivatives are not known to control signals through estrogen receptor by selectively blocking estrogen receptor.

### Objects of Invention

The main object of the present invention is to provide highly safe pharmaceutical compositions, drinks or foods for preventing the influences of endocrine disruptors.

### Summary of Invention

The present inventors have established a screening system for substances that antagonize and block endocrine disruptors in a living body upon oral intake. As a result of intensive studies, the present inventors have surprisingly found that some substances that bind to estrogen receptor in vivo do not exhibit an estrogenic activity. Thus, the present invention has been completed. The present invention is outlined as follows. The first aspect of the present invention relates to a pharmaceutical composition for treating or preventing a disease caused by binding of an agonist to estrogen receptor, which contains, as an active ingredient, a substance having an antagonistic activity against an estrogen receptor agonist.

The second aspect of the present invention relates to a drink or food for ameliorating a disease state of or preventing a disease caused by binding of an agonist to estrogen receptor, which contains, which is produced by adding thereto, and/or which is produced by diluting a substance having an antagonistic activity against an estrogen receptor agonist.

A compound, which is an antagonist that does not exhibit an estrogenic activity, selected from the group consisting of:
a compound of general formula I: 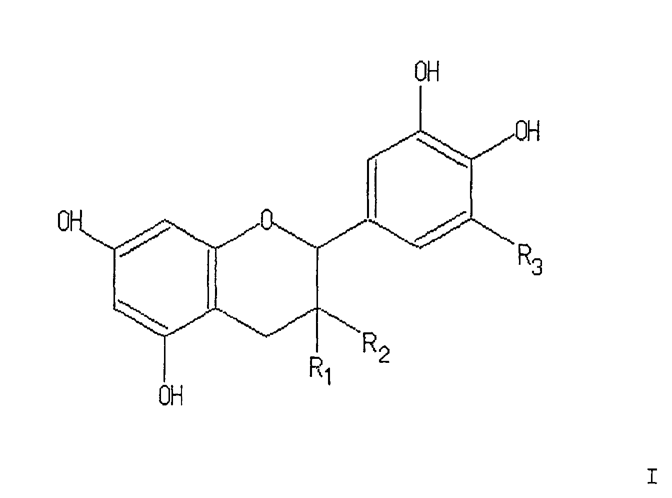
wherein R₁ and R₂ represent -H, -OH or -O-R₄; R₃ represents -H or -OH; R₄ represents a compound of formula II: 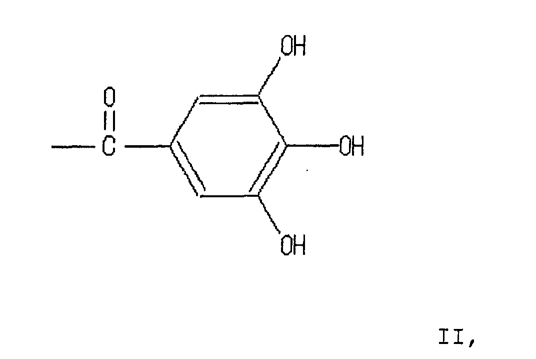 a derivative thereof or a salt thereof;
gallic acid;
a derivative thereof; and
a pharmacologically acceptable salt thereof,
is preferably used as the substance having an antagonistic activity against an estrogen receptor agonist in the first or second aspect.

The compound of formula I is exemplified by a substance belonging to catechins. For example, epicatechin, epigallocatechin, epigallocatechin gallate, epicatechin gallate, gallocatechin gallate, catechin gallate, catechin and gallocatechin can be preferably used. The derivative of gallic acid is exemplified by an ester of gallic acid. In particular, an ester of gallic acid with an alcohol having eight or more carbons is preferable according to the present invention.

The agonist according to the present invention is exemplified by an endocrine disruptor. For example, dioxins, organochlorine compounds, phenols, phthalate esters, aromatic hydrocarbons, pesticides, organotin compounds, estrogens, mirex, toxaphene, aldicarb and kepone are exemplified. More specifically, zeranol, genistein, coumestrol, o,p-DDT, kepone, methoxychlor, zearaalenone, dioxin, p-octylphenol, nonylphenol, dieldrin, butylbenzylphtalate and/or bisphenol-A are exemplified.

The third aspect of the present invention relates to an alcoholic drink containing a substance belonging to catechins, which is produced by adding thereto a substance belonging to catechins. Epigallocatechin gallate is preferably used as the substance belonging to catechins in the third aspect. The content of epigallocatechin gallate preferably ranges from 0.002 to 0. 75%(w/w). The alcoholic drink of the present invention may contain a tea extract in addition to the substance belonging to catechins. The ratio of epigallocatechin gallate to caffeine in the alcoholic drink of the present invention is preferably 0.9 or more. According to the present invention, it is now possible to provide an alcoholic drink in which the epigallocatechin gallate content is increased and the caffeine content is decreased. The alcoholic drink of the present invention has a thick feeling of tea, good amplitude and balance of taste, enhanced sweet taste of alcohol, taste tightened by bitter taste, and stimulant taste as a result of synergistic effects of the substance belonging to catechins, the tea extract and alcohol.

The fourth aspect of the present invention relates to a tea drink, which is produced by a method comprising a step of sterilization, which contains epigallocatechin gallate at a concentration of 0.01 to 0.5%(w/w), wherein the ratio of epigallocatechin gallate to caffeine is 0.9 or more. For example, the tea drink of the fourth aspect is a canned or bottled tea drink produced by a method comprising a step of sterilization. For example it is a green tea drink, a black tea drink, an oolong tea drink, a barley tea drink or a banaba tea drink.

### Brief Description of Drawings

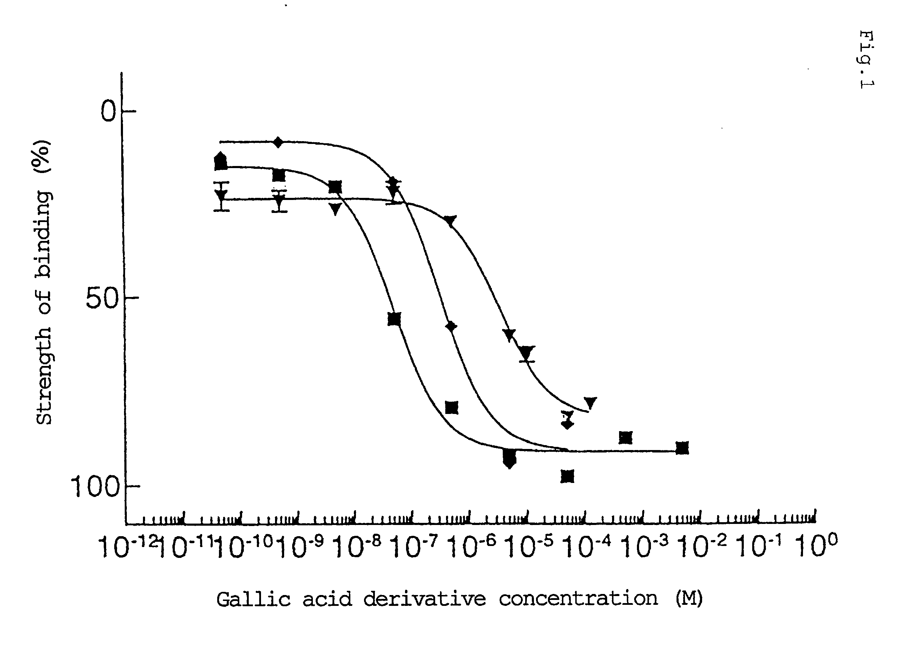

Figure 1 illustrates activities of gallic acid derivatives to bind to estrogen receptor.

### Detailed Description of the Invention

As used herein, an agonist refers to a substance that binds to estrogen receptor and exhibits an estrogenic activity. The substance that exhibits an estrogenic activity is exemplified by an endocrine disruptor or estrogen.

As used herein, an endocrine disruptor (also called an exogenous endocrine disruptor or an environmental hormone) means an exogenous substance that influences upon incorporation into a living body of an animal the normal activity of a hormone naturally exhibited in the living body. The endocrine disruptors include ones that maintain, promote or suppress the normal activity of a hormone. Substances that potentially influence the normal activity of a hormone are also included. As used herein, estrogen refers to endogenous estrogen which is normally produced in a living body, or synthetically produced estrogen. Estrogen receptor to which an agonist binds may be either of the α-type or of the β-type. Preferably, it is of the α-type.

Currently, about 70 substances (or groups of substances) are suspected to have endocrine-disrupting activities. These substances are classified in the materials for the abstract of 24th Meeting of the Japan Society for Environmental Chemistry (1998) as follows according to the analysis methods applicable to the substances: (1) Category 1: organochlorine compounds (e.g., general organochlorine compounds, polychlorinated biphenyl (PCB)); (2) Category 2: phenols (e.g., general phenols, bisphenol-A, 2,4-dichlorophenol, pentachlorophenol), (3) Category 3: phthalate esters (e.g., general phthalate esters); (4) Category 4: aromatic hydrocarbons (e.g., benzo(a)pyrene, di-2-ethylhexyl adipate (DEHA), benzophenone, 4-nitrotoluene, stylene dimer and trimer, 1,2-dibromo-3-chloropropane, styrene, n-butylbenzene); (5) Category 5: pesticides (e.g., general pesticides, 2,4,5-T (trichlorophenoxyacetic acid), 2,4-D (dichlorophenoxyacetic acid), benomyl, amitrole, methomyl); (6) Category 6: organotin compounds; (7) Category 7: estrogens; dioxins which are not classified into the above-mentioned categories; as well as mirex, toxaphene, aldicarb and kepone which are excluded from the categories. These categories and the substances are shown in Tables 1, 2 and 3.

**Table 1**

| Category/Substance | |
|---|---|
| CATEGORY 1 | Methoxychlor |
| Polychlorinated biphenyl (PCB) | Octachlorostyrene |
| Polybrominated biphenyl (PBB) | |
| Hexachlorobenzene (HCB) | CATEGORY 2 |
| Hexachlorocyclohexane | Pentachlorophenol (PCP) |
| Chlordane | Alkylphenol (C5-C9) |
| Oxychlordane | Bisphenol-A |
| trans-Nonachlor | 2,4-dichlorophenol |
| DDT | |
| DDE, DDD | CATEGORY 3 |
| Kelthane | Di-2-ethylhexyl phthalate |
| Aldrin | Butylbenzyl phtalate |
| Endrin | Di-n-butyl phthalate |
| Dieldrin | Dicyclohexyl phthalate |
| Endosulfan (benzoepin) | Diethyl phthalate |
| Heptachlor | Dipentyl phthalate |
| Heptachlor epoxide | Dihexyl phthalate |
| (continued to the right uppermost column in this table) | Dipropyl phthalate |

**Table 2**

| Category/Substance | |
|---|---|
| CATEGORY 4 | Carbaryl |
| 1,2-Dibromo-3-chloropropane | Malathion |
| Benzo(a)pyrene | Methomyl |
| Di-2-ethylhexyl adipate | Nitrophene |
| Benzophenone | Trifluralin |
| 4-Nitrotoluene | Benomyl |
| Stylene dimer and trimer | Manzeb (mancozeb) |
| Styrene | Maneb |
| n-Butylbenzene | Metiram |
| | Metribuzin |
| CATEGORY 5 | Cypermethrin |
| 2,4,5-Trichlorophenoxyacetic acid | Esfenvalerate |
| 2,4-Dichlorophenoxyacetic acid | Fenvalerate |
| Amitrole | Permethrin |
| Atrazine | Vinclozolin |
| Alachlor | Zineb |
| Simazine | Ziram |
| Ethylparathion | |
| (continued to the right uppermost column in this table) | |

**Table 3**

| Category/Substance | |
|---|---|
| CATEGORY 6 | NOT CLASSIFIED INTO THE CATEGORIES |
| Tributyltin | Dioxin |
| Triphenyltin | |
| | ENVIRONMENTAL HORMONES EXCLUDED FROM THE CATEGORIES |
| CATEGORY 7 | Mirex |
| Estradiol | Toxaphene |
| | Aldicarb |
| | Kepone (chlordecone) |

The endocrine disruptors are not limited to those listed above. For example, zeranol, genistein, coumestrol, o,p-DDT, kepone, methoxychlor, zearaalenone, dioxin, p-octylphenol, nonylphenol, dieldrin, butylbenzylphtalate and/or bisphenol-A are exemplified.

Any substance that binds to estrogen receptor, does not exhibit an estrogenic activity, and exhibits an antagonistic activity against an agonist in vivo can be preferably used as a substance having an antagonistic activity against an estrogen receptor agonist (hereinafter also referred to as an antagonist) according to the present invention. Although it is not intended to limit the present invention, for example, catechins, gallic acid and/or derivatives thereof can be preferably used.

Methods for screening agonists or antagonists are not specifically limited. For example, an in vitro screening method comprised the following steps. In the first step, candidate substances can be screened for their activities of binding to estrogen receptor according to a known method such as the method as described in Example 7. Specifically, the antagonistic activities of the candidate substances with a fluorescence-labeled ligand for binding to estrogen receptor are measured. In a second step, it is judged whether a candidate substance which has been confirmed to have an activity of binding to estrogen receptor is an agonist or an antagonist. The judgement can be conducted by determining if the substance has an estrogenic activity according to a known method. An exemplary method is the E-SCREEN assay as described in Environ. Health Perspect., 103:113-122 (1995). In the assay, a candidate substance is added to a culture of one of human breast cancer-derived cells, MCF-7, and its activity of proliferating MCF-7 is determined.

Furthermore, although it is not intended to limit the present invention, an antagonist can be effectively screened in vivo, for example, according to the method as described in Example 1. Specifically, an estrogen receptor agonist is administered, a candidate substance is further orally administered, and change in uterine weight is then determined. In this case, the determination may be carried out after increasing the absorptivity of the candidate substance upon administration by dissolving it in ethanol.

Catechins are exemplified by epicatechin, epigallocatechin, epigallocatechin gallate, epicatechin gallate, gallocatechin gallate, catechin gallate, catechin, gallocatechin and/or derivatives thereof.

Derivatives of catechins according to the present invention are not specifically limited as long as they have antagonistic activities against an estrogen receptor agonist.

Derivatives of gallic acid that can be used in the present invention include, but are not limited to, compounds each having the structure of gallic acid in the molecule. A compound such as an ester, an ether or an amide produced as a result of a reaction between the hydroxyl or carboxyl group in gallic acid and a functional group in another molecule is exemplified. In particular, an ester of the carboxyl group in gallic acid with a compound having a hydroxyl group is preferably used in the present invention.

The esters include those of gallic acid with compounds having alcoholic or phenolic hydroxyl groups. Pentyl gallate, hexyl gallate, heptyl gallate, octyl gallate, nonyl gallate, decyl gallate, undecyl gallate, dodecyl gallate tridecyl gallate, tetradecyl gallate, pentadecyl gallate, cetyl gallate, heptadecyl gallate and octadecyl gallate are exemplified. An ester of gallic acid with an aliphatic alcohol having eight or more carbons such as an ester of gallic acid with octyl alcohol, dodecyl alcohol or cetyl alcohol is particularly preferable according to the present invention. The aliphatic alcohol may have a side chain or an unsaturated hydrocarbon chain as long as the activity of antagonizing with an agonist for binding to estrogen receptor is retained. Furthermore, the hydrogen atom in the hydrocarbon chain may be substituted with halogen or a functional group such as a hydroxyl, carboxyl, amino or oxo group.

Diseases caused by binding of an agonist to estrogen receptor according to the present invention are exemplified by diseases which have been reported to be caused by abnormal response to estrogen such as decrease in sperm number, estrogen-dependent cancer, metrofibroma, endometritis, proliferation of smooth muscle cells and oligospermia. Diseases to be treated or prevented using the pharmaceutical composition of the present invention include prostatomegaly for which suppression of the functions of estrogen is expected to be therapeutically effective.

As used herein, a substance having an antagonistic activity against an estrogen receptor agonist refers to a substance that binds to estrogen receptor and exhibits an activity of antagonizing with an agonist (such as an endocrine disruptor or estrogen) binding to estrogen receptor.

According to the present invention, estrogen is exemplified by a steroid compound having an estrogenic activity such as 17β-estradiol, estrone, conjugated estrogen (Premarin (a registered trademark)), equine estrogen or 17β-ethinylestradiol.

According to the present invention, a substance having an antagonistic activity against an estrogen receptor agonist is exemplified by a compound selected from the group consisting of a compound of formula I, gallic acid, a derivative thereof and a pharmacologically acceptable salt thereof. The substances having antagonistic activities against an estrogen receptor agonist according to the present invention also include polymers and oligomers each having the structure of formula I as its basic backbone.

The derivatives are exemplified by procyanidin B-2 3,3'-digallate, procyanidin B-3, prodelphinidin B-4 3'-gallate, oolonghomobisflavan A, assamicadin A, theogallin and strictine.

Furthermore, theaflavin, theaflavin gallate, theaflavin gallate B or theaflavin digallate may be used as an active ingredient according to the present invention.

Catechins that can be used according to the present invention are exemplified by tea catechins. Catechins are a group of pigment components generically called flavonoids. Tea is known to contain eight types of catechins. Catechins to be used may be commercially available ones. Alternatively, they may be prepared according to known methods. For example, those prepared from green tea may be used. According to the present invention, methods for preparing catechins are not specifically limited. For example, they may be prepared by chemical synthesis or extraction and purification from natural products. Exemplary catechins mainly used as active ingredients according to the present invention are tea catechins. Although it is not intended to limit the present invention, they can be extracted and purified from natural products such as fermented tea (e.g., black tea), semifermented tea (e.g., oolong tea), or nonfermented tea (e.g., green tea). A tea extract can be preferably used as a substance having an antagonistic activity against an estrogen receptor agonist according to the present invention.

For example, although it is not intended to limit the present invention, an extract containing epigallocatechin gallate (hereinafter also referred to as EGCG) may be used if it is extracted from an edible material. Preferably, the EGCG content of the material is high. The EGCG-containing extracts are exemplified by tea extracts obtained using water or an organic solvent such as Sunphenon 100S, Sunphenon DCF-1, Sunphenon LA-50 and Sunphenon BG (all from Taiyo Kagaku). The EGCG contents of the above-mentioned extracts are 50, 53, 18 and 50%(w/w), respectively. The caffeine contents are 10%(w/w), below detection limit, 5%(w/w) and 3%(w/w), respectively. Intake of an excess amount of caffeine is known to lead to toxicity, carcinogenesis, elevated cholesterol level, calciuria or the like. Thus, a tea extract containing reduced amounts of impurities such as caffeine can be preferably used for drinks or foods. Furthermore, the sensory evaluation (of taste) may be improved by reducing the caffeine content.

As used herein, "tea" means a raw or dried product, with or without partial or full fermentation, of the whole plant or parts thereof (e.g., leaf, xylem, root, seed, etc.) of tea (Cammellia sinensis, (L) O. Kuntze). Each part may be used alone or any plural parts may be used in combination. Several forms of tea leaves may be used as raw materials to be extracted. For example, such forms include ones from any steps of a conventional tea production procedure such as raw tea leaves and finished tea (dried tea). In addition, the degree of fermentation is not limited. For example, grain tea such as barley tea, roasted rice tea or buckwheat tea, or leaf tea such as green tea, black tea, roasted green tea, powdered green tea, oolong tea or banaba tea may be used.

A tea extract may be used as a substance having an antagonistic activity against an estrogen receptor agonist according to the present invention if it contains a substance belonging to the tea catechins. Thus, a crude tea extract can be used. Such a crude tea extract can be obtained by extracting tea with warm (preferably hot) water or an organic solvent. For example, a lower alcohol such as methyl alcohol, ethyl alcohol, n-propyl alcohol, isopropyl alcohol or butyl alcohol, a lower ester such as methyl acetate, ethyl acetate, propyl acetate or butyl acetate, or a ketone such as acetone or methyl isobutyl ketone can be used as an organic solvent. Each organic solvent may be used alone or any plural organic solvents may be used appropriately in combination. They may be anhydrous or preferably hydrous.

A method conventionally used for extraction of a crude drug can be preferably used for the extraction with water or an organic solvent. For example, it is preferable to heat-reflux a mixture of one part by weight of (dried) tea leaves and 5 to 20 parts by weight of water or an organic solvent while stirring at a temperature of the boiling point or below. The extraction step is usually carried out for 5 minutes to 7 days, preferably 10 minutes to 24 hours. Optionally, the extraction period can be shortened by supplementary means such as stirring. Insoluble substances can be separated from the water- or organic solvent-extract by appropriate means such as filtration or centrifugation. In addition to such hot water- or organic solvent-extracts prepared according to conventional methods, products obtained by further treating the extracts with various organic solvents, adsorbents or the like are also included in the tea extracts that can be used as active ingredients for the synthesis inhibitors of the present invention. Optionally, the tea extracts may be concentrated or dried and powdered, or purified by crystallization from cold water.

The tea extract obtained as described above contains a mixture of catechins contained in tea (in particular tea leaves), i.e., tea catechins, such as epicatechin, epigallocatechin, epigallocatechin gallate, epicatechin gallate, gallocatechin gallate, catechin gallate, catechin, gallocatechin. It may additionally contain impurities derived from the raw material tea.

The pharmaceutical composition of the present invention for treating or preventing a disease caused by binding of an agonist to estrogen receptor can be produced using, as an active ingredient, a substance having an antagonistic activity against an estrogen receptor agonist.

The pharmaceutical composition for treatment or prevention of the present invention can be formulated by using a substance having an antagonistic activity against an estrogen receptor agonist to be used in the present invention as its active ingredient, and formulating it with a known pharmaceutical carrier. For the production of the composition, the substance is generally mixed with a pharmaceutically acceptable liquid or solid carrier and, optionally, solvent, dispersing agent, emulsifier, buffering agent, stabilizer, excipient, binder, disintegrant, lubricant and the like to formulate it. The formulation may be in a form of a solid preparation such as tablet, granule, powder, epipastic and capsule, or a liquid preparation such as normal solution, suspension and emulsion. In addition, it may be formulated into a dried preparation, which can be reconstituted as a liquid preparation by adding an appropriate carrier before use.

The pharmaceutical carrier can be selected according to the above-mentioned particular administration route and dosage form. For an oral preparation, starch, lactose, sucrose, mannit, carboxymethylcellulose, cornstarch, inorganic salts and the like are utilized, for example. Binder, disintegrant, surfactant, lubricant, fluidity-promoting agent, tasting agent, coloring agent, flavoring agent and the like can also be included in oral preparations. In addition, the absorptivity of a substance belonging to catechins contained in a liquid oral preparation can be made excellent, for example, by including ethanol at a concentration from 0.2 to 40%(w/w).

A parenteral preparation can be prepared according to conventional methods by dissolving or suspending a compound selected from the group consisting of a substance having an antagonistic activity against an estrogen receptor agonist, a derivative thereof and a salt thereof as an active ingredient of the pharmaceutical composition of the present invention in a diluent. The diluents include injectable distilled water, physiological saline, aqueous glucose solution, injectable vegetable oil, sesame oil, peanut oil, soybean oil, corn oil, propylene glycol and polyethylene glycol. Optionally, sterilizer, stabilizer, osmotic regulator, smoothing agent and the like may be added to the solution or suspension.

The pharmaceutical composition for treatment or prevention of the present invention is administered through a suitable route for the dosage form of the composition. The administration route is not limited to a specific one. The composition can be administered internally or externally (or topically) or by injection. The injectable preparation can be administrated intravenously, intramuscularly, subcutaneously, intradermally and the like, for example. External preparations include a suppository.

A dosage of the pharmaceutical composition for treatment or prevention of the present invention is appropriately determined and varies depending on the particular dosage form, administration route and purpose as well as age, weight and conditions of a patient to be treated. In general, a daily dosage contained in the formulation of the substance having an antagonistic activity against an estrogen receptor agonist to be used in the present invention is, based on the nontoxic administration level, from about 0.001 g to about 100 g, preferably from about 0.001 g to about 10 g, more preferably from about 0.001 g to about 5 g. Of course, the dosage can vary depending on various factors. Therefore, in some cases, a less dosage than the above may be sufficient but, in other cases, a dosage more than the above may be required. The pharmaceutical composition of the present invention can be administrated orally as it is, or it can be taken daily by adding to selected foods or drinks such as alcoholic drinks or tea drinks. The substance having an antagonistic activity against an estrogen receptor agonist to be used in the present invention may be used as a raw material for a drink or food for ameliorating a disease state of or preventing a disease caused by binding of an agonist to estrogen receptor.

The substance having an antagonistic activity against an estrogen receptor agonist according to the present invention to be added to a drink or food may be in any form such as liquid, paste, powder, flake or granule. It is preferably dried and in a form of powder, flake or granule in view of easy handling or mixing with other additives. Drying can be carried out according to a conventional drying method such as spray drying, drum drying, tray drying, vacuum drying or freeze drying.

The drink or food of the present invention contains a substance having an antagonistic activity against an estrogen receptor agonist. It contains, is produced by diluting, and/or is produced by adding thereto the substance.

The content of the substance having an antagonistic activity against an estrogen receptor agonist in the drink or food of the present invention is not specifically limited. A drink or food that contains the substance at a concentration from 0.0005 to 90%(w/w) is exemplified.

The foods or drinks of the present invention are not limited to specific ones and examples thereof include the following: products of processed cereal (e.g., wheat flour product, starch product, premixed product, noodle, macaroni, bread, bean jam, buckwheat noodle, wheat-gluten bread, rice noodle, gelatin noodle and packed rice cake), products of processed fat and oil (e.g., plastic fat and oil, tempura oil, salad oil, mayonnaise and dressing), products of processed soybeans (e.g., tofu, miso and fermented soybean), products of processed meat (e.g., ham, bacon, pressed ham and sausage), processed marine products (e.g., frozen ground fish, boiled fish paste, tubular roll of boiled fish paste, cake of ground fish, deep-fried patty of fish paste, fish ball, sinew, fish meat ham or sausage, dried bonito, product of processed fish egg, canned marine product and fish boiled in sweetened soy sauce), dairy products (e.g., raw milk, cream, yogurt, butter, cheese, condensed milk, powdered milk and ice cream), products of processed vegetables and fruits (e.g., paste, jam, pickle, fruit juice, vegetable drink and mixed drink), confectioneries (e.g., chocolate, biscuit, sweet bun, cake, rice-cake sweet and rice sweet), alcohol drinks (e.g., sake, Chinese liquor, wine, whisky, shochu, vodka, brandy, gin, rum, beer, soft alcohol drink, fruit liquor and liqueur), luxury drinks (e.g., green tea, black tea, oolong tea, coffee, soft drink and lactic acid drink), seasonings (e.g., soy sauce, sauce, vinegar and sweet sake), canned, bottled or bagged foods (e.g., various cooked foods such as rice topped with cooked beef and vegetables, rice boiled together with meat and vegetables in a small pot, steamed rice with red beans, and curry), semi-dried or condensed foods (e.g., liver paste, other spreads, soup for buckwheat noodle or udon and condensed soup), dried foods (e.g., instant noodle, instant curry, instant coffee, powdered juice, powdered soup, instant miso soup, cooked food, cooked drink and cooked soup), frozen foods (e.g., sukiyaki, chawan-mushi, grilled eel, hamburger steak, shao-mai, dumpling stuffed with minced pork, various stick-shaped foods and fruit cocktail), solid or liquid foods (e.g., soup), processed agricultural or forest products (e.g., spice), processed livestock products, processed marine products and the like.

There is no particular limitation for the method for producing a drink or food of the present invention. Any processes including cooking, processing and other generally employed processes for producing drinks or foods can be used as long as the resultant drink or food contains, is produced by adding thereto, and/or is produced by diluting the substance having an antagonistic activity against an estrogen receptor agonist as an active ingredient. For example, a functional drink or food having a novel flavor and a high EGCG content can be produced by mixing EGCG-containing powder (Sunphenon 100S) with green tea at an appropriate ratio. The concentration of tea is usually 0.05 to 4, preferably 0.1 to 2 in view of flavor defining the concentration used for drinking as 1 (standard). A sweetener, an acidulant, a flavor and the like can be optionally added to the resultant EGCG-containing drink or food. A reducing agent may be added.

For example, ethanol at a concentration of 0.5 to 20%(w/w) may be added to the drink to prepare an alcoholic drink containing EGCG. Carbon dioxide may be further introduced to prepare a carbonated alcoholic drink containing EGCG.

The present invention provides a drink or food in which the EGCG content is increased. The EGCG content in such a drink or food is preferably from 0.002 to 5%(w/w), more preferably from 0.01 to 0.5%(w/w). EGCG may be added in a tea extract or an extract containing catechins. Alternatively, purified EGCG may be added.

An EGCG-containing drink or food in which caffeine is decreased or from which caffeine is removed and which does not result in intake of an excess amount of caffeine is provided by adding EGCG to a drink or food to increase the EGCG content.

The present invention provides an alcoholic drink which is produced by adding thereto a substance belonging to catechins. As used herein, an alcoholic drink refers to a drink that contains ethanol. The content of ethanol in an alcoholic drink is, for example, from 0.2 to 40%(w/w).

The alcohol drink is not specifically limited as long as it contains a substance belonging to catechins being added. Sake, Chinese liquor, wine, whisky, shochu, vodka, brandy, gin, rum, beer, soft alcohol drink, fruit liquor and liqueur each containing a substance belonging to catechins being added are exemplified. The content of the substance belonging to catechins is increased, and the amount of caffeine relative to the substance is decreased in such an alcoholic drink as compared with the corresponding alcoholic drink containing a tea extract.

The absorptivity of a substance belonging to catechins is increased in the presence of alcohol. Therefore, the absorptivity of the substance belonging to catechins in the alcoholic drink of the present invention is very high. Since it is now possible to increase only the content of a substance belonging to catechins, selective intake of the substance is possible. If a large amount of a tea extract is taken, a large amount of caffeine is also taken. However, if a substance belonging to catechins is taken in the alcoholic drink of the present invention, the intake of a large amount of caffeine is avoided. Thus, the alcoholic drink of the present invention is an excellently safe alcoholic drink for selective intake of a substance belonging to catechins, which is useful for improving health.

The content of a substance belonging to catechins in the alcoholic drink of the present invention is preferably from 0.004 to 1.5%(w/w), more preferably from 0.02 to 1%(w/w) in view of sensory evaluation. The content of EGCG in the alcoholic drink of the present invention is preferably from 0.002 to 0.75%(w/w), more preferably from 0.01 to 0.5%(w/w) in view of sensory evaluation.

A tea drink (e.g., a black tea drink, a green tea drink, an oolong tea drink or a barley tea drink) that contains EGCG at a concentration of 0.01 to 0.5%(w/w), wherein the ratio of EGCG to caffeine is 0.9 or more is a tea drink having a novel flavor in which the flavor of tea extract is enhanced and the caffeine content is decreased. As used herein, a tea drink refers to a drink that is produced using tea as the principal raw material. It is produced by a method comprising a step of sterilization. The tea drink is sterilized, for example, by heating.

A substance having an antagonistic activity against an estrogen receptor agonist may be added before, during or after the cooking or processing. The substance may be diluted by adding thereto a cooked or processed product, or a material for the cooked or processed product. The substance may be added during any one of steps of production of drinks or foods. The substance may be diluted by adding thereto a drink or food, or a material therefor, to include it into the drink or food. The substance may be added once or several times. Then, a novel drink or food can be conveniently produced. Such a drink or food contains an effective amount of a substance having an antagonistic activity against an estrogen receptor agonist. Regardless of the production steps selected, a drink or food which contains, which is produced by adding thereto, and/or which is produced by diluting a substance having an antagonistic activity against an estrogen receptor agonist, is defined as the food or drink of the present invention.

The drink or food of the present invention contains, is produced by adding thereto, and/or is produced by diluting a substance having an antagonistic activity against an estrogen receptor agonist. Such a drink or food may be concentrated. The form of the drink or food is not specifically limited as long as a substance having an antagonistic activity against an estrogen receptor agonist is contained in an amount necessary for exhibiting its physiological function. The food or drink of the present invention may be in an edible form such as tablet, granule and capsule.

A concentrated drink or food which is provides as a concentrated product and used after appropriate dilution before intake is included in the drinks or foods (including alcoholic drinks and tea drinks) of the present invention.

When the pharmaceutical composition for treatment or prevention of the present invention is administered, the antagonist contained in the pharmaceutical composition antagonizes with a substance having an endocrine-disrupting activity for its binding. Thus, the harmful influence of the substance on a living body can be treated or prevented. The pharmaceutical composition of the present invention can be used to ameliorate or prevent an inappropriate physiological disease state due to estrogen by antagonism of estrogen. Furthermore, as demonstrated in Examples below, enlargement of uterine caused by a substance having an endocrine-disrupting activity can be prevented according to the present invention. Thus, the pharmaceutical composition for treatment or prevention of the present invention is useful for the treatment or the prevention of various disorders or diseases due to abnormal biological control involving estrogen.

When the drink or food of the present invention is taken, the antagonist contained in the drink or food antagonizes with a substance having an endocrine-disrupting activity. Then, the harmful influence of the substance on a living body can be treated or prevented. Furthermore, an inappropriate physiological disease state due to estrogen can be ameliorated or prevented by antagonism of estrogen by the drink or food of the present invention.

### Examples

The following examples further illustrate the present invention in detail but are not to be construed to limit the scope thereof.

### Example 1

### Examination of anti-estrogenic activity of EGCG against bisphenol-A

### (1) Influence of EGCG on immature rat uterine weight

A 17-days old female SD rat (purchased from Japan SLC) was received together with its mother, weaned at 20 days of age, and subjected to experiments from the next day. Five ml per kg body weight of EGCG (Sigma) or bisphenol-A (hereinafter referred to as BisA, Tokyo Kasei Kogyo) suspended in peanut oil (Nacalai Tesque) was administered orally and forcibly. Peanut oil was administered to a control rat in a similar manner. Administration was carried out once a day for three successive days. The uterine removed 24 hours after the last administration was weighed.

### (2) Examination of agonistic (estrogenic) activity

BisA or EGCG (80 or 800 mg/kg) was administered. The results are shown in Table 4. When 800 mg/kg of BisA was administered, the uterine weight was increased to 137% of that of the control group. On the other hand, Administration of EGCG did not result in increased uterine weight.

**Table 4**

| Group | (n) | Uterine weight (mg) | Ratio (%) |
|---|---|---|---|
| Control | 7 | 40.4±2.8 | 100 |
| BisA 800 mg/kg | 7 | 55.2±2.3** | 137 |
| EGCG 80 mg/kg | 7 | 42.2±1.7 | 104 |
| 800 mg/kg | 6 | 39.3±3.2 | 97 |

| | | | |
|---|---|---|---|
| Mean ± standard error **:p<0.01 vs control | | | |

### (3) Examination of antagonistic (anti-estrogenic) activity

BisA (800 mg/kg) and EGCG (800 mg/kg) were administered at the same time. The results are shown in Table 5.

When 800 mg/kg of BisA was administered, the uterine weight was increased to 147% of that of the control group (100%). On the other hand, when EGCG was additionally administered, the increase was suppressed to 116%.

**Table 5**

| Group | (n) | Uterine weight (mg) | Ratio (%) |
|---|---|---|---|
| Control | 5 | 37.2±2.4 | 100 |
| BisA 800 mg/kg | 4 | 54.7±5.5* | 147 |
| BisA + EGCG 800 mg/kg | 6 | 43.0±2.7 | 116 |

| | | | |
|---|---|---|---|
| Mean ± standard error *:p<0.05 vs control | | | |

### Example 2

### Examination of anti-estrogenic activity of solution of EGCG in ethanol against bisphenol-A

A 17-days old female SD rat (purchased from Japan SLC) was received together with its mother, weaned at 20 days of age, and subjected to experiments from the next day. BisA was weighed such that it would be administered at 200 or 40 mg/kg and dissolved in a small volume of ethanol. Peanut oil was added thereto to result in a final ethanol concentration of 5%. The resulting mixture was orally administered for three successive days. The uterine removed on the next day was weighed. EGCG was dissolved by adding ethanol and water was then added thereto to adjust the final ethanol concentration to 10%. The solution was orally administered on the day before the administration of BisA and 30 minutes before each of the successive administrations of BisA. The results are shown in Table 6.

**Table 6**

| Group | (n) | Uterine weight (mg) | Ratio (%) |
|---|---|---|---|
| Control | 5 | 43.4±2.7 | 100 |
| BisA 200 mg/kg | 6 | 57.7±5.2* | 133 |
| BisA 200 mg/kg + EGCG 100 mg/kg | 5 | 47.4±2.6 | 109 |
| BisA 200 mg/kg + EGCG 200 mg/kg | 5 | 39.8±4.5 | 92 |
| BisA 40 mg/kg | 4 | 64.5±9.2* | 149 |
| BisA 40 mg/kg + EGCG 100 mg/kg | 5 | 41.2±2.2 | 95 |

| | | | |
|---|---|---|---|
| Mean ± standard error *:p<0.05 vs control | | | |

When 200 mg/kg of BisA was administered, the uterine weight was increased to 133% of that of the control group (100%). On the other hand, when 100 mg/kg or 200 mg/kg of EGCG was additionally administered, the increase was suppressed to 109% or 92%, respectively. Furthermore, when 40 mg/kg of BisA was administered, the uterine weight was increased to 149% of that of the control group (100%). On the other hand, when 100 mg/kg of EGCG was additionally administered, the increase was suppressed to 95%.

It was demonstrated that when EGCG was orally administered, it did not exhibit an estrogenic activity (i.e., it did not act as an agonist), but acted as an antagonist against BisA. Furthermore, it was demonstrated that EGCG dissolved with the addition of an appropriate amount of ethanol suppressed the increase in uterine weight even if a relatively small amount was administered. A pharmaceutical composition, a drink or a food for preventing or ameliorating the disease state in an individual suffering from an inappropriate physiological disease state due to an endocrine disruptor (e.g., decrease in sperm number or prostatomegaly), or deteriorated tumor, endometritis or metrofibroma due to an abnormal estrogenic activity can be provided using EGCG as an antagonist against estrogen.

### Example 3

### Examination of anti-estrogenic activity of solution of EGCG in ethanol against 4-nonylphenol

A 16-days old female SD rat (Japan SLC) was purchased together with its mother, weaned at 19 days of age, and subjected to experiments from the next day.

4-Nonylphenol (hereinafter referred to as NP) was weighed such that it would be administered at 50 mg/kg and diluted by adding peanut oil thereto. The resulting mixture was orally administered for three successive days. The uterine removed on the day after the third administration day was weighed. EGCG was dissolved by adding ethanol and water was then added thereto to adjust the final ethanol concentration to 10%. The solution was orally administered 90 minutes before the administration of NP. The results are shown in Table 7.

**Table 7**

| Group | (n) | Uterine weight (mg) | Ratio (%) |
|---|---|---|---|
| Control | 7 | 30.9±2.1 | 100 |
| NP 50 mg/kg | 7 | 44.4±3.2* | 144 |
| NP 50 mg/kg + EGCG 200 mg/kg | 7 | 33.7±1.9 | 109 |

| | | | |
|---|---|---|---|
| Mean ± standard error *:p<0.05 vs control | | | |

As a result, when 50 mg/kg of NP was administered, the uterine weight was increased to 144% of that of the control group (100%). On the other hand, when 200 mg/kg of EGCG was additionally administered, the increase was suppressed to 109%.

### Example 4

### Examination of effect of ethanol concentration on anti-estrogenic activity of EGCG

A 16-days old female SD rat (Japan SLC) was purchased together with its mother, weaned at 19 days of age, and subjected to experiments from the next day.

BisA was weighed such that it would be administered at 40 mg/kg and dissolved in a small volume of ethanol. Peanut oil was added thereto to result in a final ethanol concentration of 5%. The resulting mixture was subcutaneously administered for three successive days. The uterine removed on the day after the third administration day was weighed. EGCG (200 mg/kg) was dissolved by adding ethanol and water was then added thereto to adjust the final ethanol concentration to 10, 5, 1, 0.5 or 0%. The solution was orally administered 90 minutes before the administration of BisA. The results are shown in Table 8.

When 40 mg/kg of BisA was administered, the uterine weight was increased to 186% of that of the control group (100%). When 200 mg/kg of EGCG was additionally administered without ethanol, the increase was suppressed only to 161%. On the other hand, when ethanol was included at a concentration of 5% or 10%, the increase was suppressed to 140% or 138%, respectively. Thus, the increase in uterine weight was significantly suppressed as compared with the case where ethanol was not included.

**Table 8**

| Group | (n) | Uterine weight (mg) | Ratio (%) |
|---|---|---|---|
| Control | 7 | 26.9 ± 2.3 | 100 |
| BisA 40 mg/kg | 7 | 49.9 ± 2.1* | 186 |
| BisA + EGCG in 10 % ethanol | 7 | 37.0 ± 1.3* | 138** |
| BisA + EGCG in 5 % ethanol | 7 | 37.6 ± 1.8* | 140** |
| BisA + EGCG in 1 % ethanol | 7 | 40.1 ± 2.0* | 149 |
| BisA + EGCG in 0.5% ethanol | 7 | 40.9 ± 1.8* | 152 |
| BisA + EGCG in 0 % ethanol | 7 | 43.3 ± 1.8* | 161 |

| | | | |
|---|---|---|---|
| Mean ± standard error *:p<0.05 vs control | | | |
| **:p<0.05 vs ethanol 0% | | | |

According to the results, it is expected that, also in case of subcutaneous administration of BisA, administration of EGCG in ethanol at a concentration of 5% or more would result in a stronger anti-estrogenic activity than that observed for EGCG administration without ethanol.

### Example 5

### Examination of anti-estrogenic activity of solution of EGCG in ethanol

A 16-days old female SD rat (Japan SLC) was purchased together with its mother, weaned at 19 days of age, and subjected to experiments from the next day.

BisA was weighed such that it would be administered at 40 mg/kg and dissolved in a small volume of ethanol. Peanut oil was added thereto to result in a final ethanol concentration of 5%. The resulting mixture was subcutaneously administered for three successive days. The uterine removed on the day after the third administration day was weighed. EGCG (200 mg/kg) was dissolved by adding ethanol and water was then added thereto to adjust the final ethanol concentration to 10 or 0%. The solution was orally administered 30 minutes before the administration of BisA. The results are shown in Table 9.

When 40 mg/kg of BisA was administered, the uterine weight was increased to 162% of that of the control group (100%). When 200 mg/kg of EGCG was additionally administered without ethanol, the increase was not suppressed. On the other hand, when EGCG was administered in 10% ethanol solution, the increase was suppressed to 118%.

**Table 9**

| Group | (n) | Uterine weight (mg) | Ratio (%) |
|---|---|---|---|
| Control | 6 | 30.8 ± 3.1 | 100 |
| BisA 40mg/kg | 6 | 50.0 ± 2.7* | 162 |
| BisA + EGCG in 10% ethanol | 7 | 36.4 ± 1.4* | 118** |
| BisA + EGCG in 0% ethanol | 7 | 50.9 ± 2.2* | 165 |

| | | | |
|---|---|---|---|
| Mean ± standard error *:p<0.05 vs control | | | |
| **:p<0.05 vs ethanol 0% | | | |

### Example 6

Dose dependency of anti-estrogenic activity of solution of EGCG in ethanol

A 16-days old female SD rat (Japan SLC) was purchased together with its mother, weaned at 19 days of age, and subjected to experiments from the next day. BisA was weighed such that it would be administered at 40 mg/kg and dissolved in a small volume of ethanol. Peanut oil was added thereto to result in a final ethanol concentration of 5%. The resulting mixture was subcutaneously or orally administered for three successive days. The uterine removed on the day after the third administration day was weighed. EGCG was dissolved by adding ethanol and water was then added thereto to adjust the final ethanol concentration to 10%. The solution was orally administered 30 minutes before the administration of BisA. The results are shown in Table 10.

When 40 mg/kg of BisA was orally administered, the uterine weight was increased to 140% of that of the control group (100%). On the other hand, when EGCG was additionally administered in a solution containing 10% ethanol, the increase was suppressed to 106% even if as little as 40 mg/kg of EGCG was administered. Furthermore, when 40 mg/kg of BisA was subcutaneously administered, the uterine weight was increased to 160% of that of the control group. On the other hand, the increase was suppressed to 136% by the administration of the EGCG solution (40 mg/kg).

**Table 10**

| Group | (n) | Urine weight (mg) | Ratio (%) |
|---|---|---|---|
| Control | 6 | 32.7 ± 1.0 | 100 |
| BisA 40 mg/kg, oral | 6 45.7 ± 2.3* | | 140 |
| BisA + EGCG 40 mg/kg | 6 | 34.8 ± 2.8 | 106** |
| BisA + EGCG 100 mg/kg | 5 | 33.0 ± 4.4 | 101** |
| BisA 40 mg/kg, subcutaneous | 5 | 52.2 ± 2.9* | 160 |
| BisA + EGCG 40 mg/kg | 5 | 44.4 ± 1.5* | 136** |
| BisA + EGCG 100 mg/kg | 5 | 39.6 ± 1.0* | 121*** |

| | | | |
|---|---|---|---|
| Mean ± standard error *:p<0.05 vs control | | | |
| **:p<0.05 vs BisA 40 mg/kg, oral | | | |
| ***:p<0.05 vs BisA 40 mg/kg, subcutaneous | | | |

### Example 7

Bindings of various gallic acid derivatives to estrogen receptor were tested using FP Screen-for-Competitors kit, ER-α, high sensitivity kit (V2577: PanVera). The tests were carried out basically according to the procedure as described in the manual attached to the kit. One of various gallic acid derivatives (n-octyl gallate (gallic acid n-octyl ester), n-dodecyl gallate (gallic acid n-dodecyl ester) and n-cetyl gallate (gallic acid n-hexadecyl ester)) was dissolved in DMSO and serially diluted to prepare diluted samples (0.5 M - 0.0000000005 M). 49 µL of Screening buffer attached to the kit was added to 1 µL of one of the diluted samples and stirred thoroughly. 50 µL of 10 nM-15 nM:ES1-ER(α) complex (a complex of ES1 (a fluorescent ligand that binds to ER) and ER(α) (estrogen receptor α)) attached to the kit was added thereto, resulting in the total volume of 100 µL. After allowing to stand for 1 hour at room temperature, fluorescence polarization was measured at an excitation wavelength of 360 nm and an emission wavelength of 535 nm using Full-Range BEACON 2000 (PanVera).

The results are shown in Figure 1 and Table 11. In the figure, squares ( ■ ), triangles ( ▼ ) and diamonds ( ◆ ) represent the results for n-cetyl gallate, n-octyl gallate and n-dodecyl gallate, respectively. The strength of binding of each gallic acid derivative to estrogen receptor was expressed as the concentration at which the derivative exhibited an activity of inhibiting 50% of the binding of the ES1-ER(α) complex by competing with ES1 in the complex (IC₅₀). It was demonstrated that the gallic acid derivatives (n-octyl gallate, n-dodecyl gallate and n-cetyl gallate) had activities of binding to estrogen receptor although the strength of binding varied.

**Table 11**

| Sample | IC₅₀ (M) |
|---|---|
| n-Octyl gallate | 3.38 x 10⁻⁶ |
| n-Dodecyl gallate | 3.11 x 10⁻⁷ |
| n-Cetyl gallate | 4.76 x 10⁻⁸ |

### Example 8

A test for proliferation of cultured cells was carried out according to a method called the E-SCREEN assay as described in Ana M. Soto et al. (Environ. Health Perspect. 103:113-122(1995)). Briefly, 4.0 x 10³ of human breast cancer-derived MCF-7 cells in a volume of 100 µL were seeded into each well of a 96-well plate. The plate was incubated overnight. BisA or a combination of BisA and n-octyl gallate was then added thereto. A well without the addition was used as a control. The cells were further cultured for 4 days in a medium, Dulbecco's modified Eagle medium without Phenol Red supplemented with 5% FCS treated with activated carbon-dextran. The MTT method was used to measured the cell numbers.

The results are shown in Table 12. The number of the cells proliferated in the presence of BisA was 1.8-fold more than that observed for the control. On the other hand, it was demonstrated that when BisA and n-octyl gallate were added, the proliferation of the cells was suppressed to a degree almost the same as that of the control.

**Table 12**

| Additive | Relative rate of increase in cell number (%) |
|---|---|
| No addition (control) | 100 |
| BisA | 180 |
| BisA + n-octyl gallate | 91 |

### Example 9

### Anti-estrogenic activity of n-octyl gallate against BisA

A 16-days old female SD rat (Japan SLC) was purchased together with its mother, weaned at 19 days of age, and subjected to experiments from the next day. n-Octyl gallate (Tokyo Kasei Kogyo) was dissolved in a small volume of ethanol. Peanut oil was added thereto to result in a final ethanol concentration of 5%. The resulting mixture was orally administered to the 20-days old female SD rat for three successive days. The uterine removed on the day after the last administration day was weighed. Peanut oil containing 5% ethanol was used for a vehicle administration group. BisA was dissolved in a small volume of ethanol. Peanut oil was added thereto to result in a final ethanol concentration of 5%. It was orally administered 1.5 hours after the administration of n-octyl gallate. The results are shown in Table 13.

When 40 mg/kg of BisA was administered, the uterine weight was increased to 161% of that of the control group (100%). On the other hand, when 126 or 25 mg/kg of n-octyl gallate was additionally administered, the increase was significantly suppressed to 108%, i.e., equivalent to the control.

**Table 13**

| Group | (n) | Uterine weight (mg) | Ratio (%) |
|---|---|---|---|
| Control | 5 | 30.4 ± 0.7 | 100 |
| BisA 40 mg/kg + vehicle | 7 | 48.9 ± 2.1* | 161 |
| BisA + n-octyl gallate 25 mg/kg | 6 | 32.7 ± 3.5 | 108** |
| BisA + n-octyl gallate 126 mg/kg | 7 | 32.9 ± 3.6 | 108** |

| | | | |
|---|---|---|---|
| Mean ± standard error *:p<0.05 vs control | | | |
| **:p<0.05 vs vehicle | | | |

n-Octyl gallate exhibited an anti-estrogenic activity against BisA. The effect was equivalent to or stronger than that of EGCG when compared taking the dosage into consideration.

### Example 10

### Anti-estrogenic activity of n-octyl gallate against NP

A 16-days old female SD rat (Japan SLC) was purchased together with its mother, weaned at 19 days of age, and subjected to experiments from the next day. n-Octyl gallate (Tokyo Kasei Kogyo) was dissolved in a small volume of ethanol. Peanut oil was added thereto to result in a final ethanol concentration of 5%. The resulting mixture was orally administered to the 20-days old female SD rat for three successive days. The uterine removed on the day after the last administration day was weighed. Peanut oil containing 5% ethanol was used for a vehicle administration group. NP weighed such that it would be administered at 50 mg/kg was diluted by adding peanut oil. It was orally administered 1.5 hours after the administration of n-octyl gallate. The results are shown in Table 14.

When 50 mg/kg of NP was administered, the uterine weight was increased to 144% of that of the control group (100%). On the other hand, when 126 mg/kg of n-octyl gallate was additionally administered, the increase was significantly suppressed to 117%.

**Table 14**

| Group | (n) | Uterine weight (mg) | Ratio (%) |
|---|---|---|---|
| Control | 7 | 33.1 ± 1.2 | 100 |
| NP 50 mg/kg + vehicle | 7 | 47.6 ± 2.3* | 144 |
| NP + n-octyl gallate 25 mg/kg | 5 | 40.4 ± 3.6 | 122 |
| NP + n-octyl gallate 126 mg/kg | 5 | 38.6 ± 2.4 | 117** |

| | | | |
|---|---|---|---|
| Mean ± standard error *:p<0.05 vs control | | | |
| **:p<0.05 vs vehicle | | | |

n-Octyl gallate exhibited an anti-estrogenic activity also against NP. The activity was equivalent to or stronger than that of EGCG when compared taking the dosage into consideration.

### Example 11

### Anti-estrogenic activity of n-octyl gallate or n-cetyl gallate against subcutaneously administered BisA

A 17-days old female SD rat (Japan SLC) was purchased together with its mother, weaned at 20 days of age, and subjected to experiments from the next day. n-Octyl gallate or n-cetyl gallate (both from Tokyo Kasei Kogyo) was dissolved in a small volume of ethanol. Peanut oil was added thereto to result in a final ethanol concentration of 5%. The resulting mixture was orally administered to the 21-days old female SD rat for three successive days. The uterine removed on the day after the last administration day was weighed. Peanut oil containing 5% ethanol was used for a vehicle administration group. BisA was dissolved in a small volume of ethanol. Peanut oil was added thereto to result in a final ethanol concentration of 5%. The mixture was subcutaneously administered. The results are shown in Table 15.

When 40 mg/kg of BisA was administered, the uterine weight was increased to 145% of that of the control group (100%). On the other hand, when 5 or 25 mg/kg of n-octyl gallate was additionally administered, the increase was significantly suppressed to 117 or 102%, respectively. Furthermore, when 1 or 5 mg/kg of n-cetyl gallate was additionally administered, the increase was significantly suppressed to 109 or 104%, respectively, i.e., equivalent to the control in each case.

**Table 15**

| Group | (n) | Uterine weight (mg) | Ratio (%) |
|---|---|---|---|
| Control | 7 | 41.3 ± 3.7 | 100 |
| BisA 40 mg/kg + vehicle | 7 | 59.7 ± 2.7* | 145 |
| BisA + n-octyl gallate 5 mg/kg | 7 | 48.3 ± 1.5 | 117** |
| BisA + n-octyl gallate 25 mg/kg | 7 | 42.1 ± 1.9 | 102** |
| BisA + n-cetyl gallate 1 mg/kg | 7 | 45.1 ± 2.3 | 109** |
| BisA + n-cetyl gallate 5 mg/kg | 7 | 43.1 ± 2.0 | 104** |

| | | | |
|---|---|---|---|
| Mean ± standard error *:p<0.05 vs control | | | |
| **:p<0.05 vs vehicle | | | |

n-Octyl gallate and n-cetyl gallate exhibited anti-estrogenic activities against BisA.

### Example 12

Sunphenon DCF-1 as an EGCG-containing extract was added to commercially available barley tea at a concentration of 0.001, 0.01, 0.05, 0.1, 0.5, 1.0 or 1.5%. The resulting products were subjected to sensory evaluation. The panel consisted of 10 members. Sensory evaluation for taste, flavor, color and total evaluation was conducted by comparing the barley tea drink of the present invention which contains the EGCG-containing extract being added and a control barley tea without the addition. Five grades were used for the sensory evaluation (1: good; 5: bad). The results were expressed using mean values. The results are shown in Table 16.

**Table 16:**

| Effect of addition of EGCG-containing extract | | | | | |
|---|---|---|---|---|---|
| EGCG-containing extract (%) | Sensory evaluation | | | | |
| | Taste | Flavor | Color | Total | Evaluation |
| 0 (control) | 4.0 | 3.5 | 2.8 | 3.8 | Normal |
| 0.001 | 3.6 | 3.3 | 2.8 | 3.5 | Slightly good balance of taste Weak effect of tightening taste |
| 0.004 | 3.3 | 3.2 | 2.8 | 3.3 | Amplitude of taste Effect of tightening taste Balance of taste |
| 0.02 | 3.2 | 3.0 | 2.8 | 3.1 | Amplitude of taste Effect of tightening taste Good balance of taste |
| 0.05 | 3.2 | 3.0 | 2.8 | 3.1 | Slight stimulant taste Thick feeling |
| 0.1 | 3.0 | 3.0 | 2.8 | 3.0 | Stimulant taste Thick feeling |
| 0.5 | 3.1 | 3.0 | 2.8 | 3.0 | Stimulant taste Thick feeling |
| 1.0 | 3.2 | 3.0 | 2.8 | 3.1 | Strong stimulant taste Good amplitude and balance Thick feeling |
| 1.5 | 3.4 | 3.0 | 2.8 | 3.3 | Strong stimulant taste Good amplitude and balance Thick feeling |
| 2.0 | 3.6 | 3.2 | 2.8 | 3.5 | Balance of taste spoiled by stimulant taste Thick feeling |

As shown in Table 16, the products containing the EGCG-containing extract at concentrations ranging from 0.02 to 1.0%(w/w) were evaluated to have good thick feeling, amplitude and balance of taste and to be preferable. The total evaluation score for each of the products containing the EGCG-containing extract at concentrations ranging from 0.004 to 1.5%(w/w) was 3.3 or less, indicating that these products were evaluated to be preferable.

### Example 13

Ethanol was added to the barley tea drink prepared in Example 12 (containing Sunphenon DCF-1 as an EGCG-containing extract at a concentration of 0.05%(w/v)) at a final concentration of 1, 2, 4, 8, 16, 20 or 35%(v/v) to prepare a tea drink that contained ethanol and the EGCG-containing extract. Sensory evaluation was conducted as described in Example 12. The results are shown in Table 17.

**Table 17:**

| Effect of addition of EGCG-containing extract | | | | | |
|---|---|---|---|---|---|
| Alcohol (%, v/v) | Sensory evaluation | | | Total | Evaluation |
| | Taste | Flavor | Color | | |
| 0 | 3.2 | 3.0 | 3.0 | 3.1 | Amplitude |
| | | | | | Good balance of taste |
| 2 | 2.8 | 2.7 | 2.6 | 2.7 | Mild flavor and taste |
| | | | | | Enhanced bitter taste |
| 4 | 2.5 | 2.6 | 2.5 | 2.5 | Mild flavor and taste |
| | | | | | Enhanced flavor |
| | | | | | Enhanced bitter taste |
| 8 | 2.2 | 2.6 | 2.5 | 2.4 | Same as the above |
| | | | | | |
| 16 | 2.6 | 2.7 | 2.6 | 2.6 | Same as the above |
| | | | | | |
| 20 | 3.0 | 2.7 | 2.7 | 2.7 | Slightly noticeable alcoholic smell |
| | | | | | Depth of taste |
| 35 | 3.0 | 2.8 | 2.7 | 2.8 | Noticeable alcoholic smell |
| | | | | | Depth of taste |

As shown in Table 17, the tea drinks each containing ethanol at a concentration of 2 to 35%(v/v) and EGCG were generally evaluated to have mild flavor and taste, smooth texture, enhanced bitter taste and prominent tea flavor as compared with the product without alcohol. Thus, a synergistic effect of improving flavor and taste by the combination of EGCG and alcohol was observed. The total evaluation score for each of the products containing ethanol at concentrations ranging from 2 to 35%(v/v) was 2.8 or less, indicating that these products were evaluated to be preferable.

The present invention provides a tasty alcoholic drink with a high EGCG content having a synergistic effect of improving flavor and taste by the combination of EGCG and alcohol.

### Example 14

(1) Tea drinks each containing an EGCG-containing extract and having the composition as shown in Table 18 were prepared using Sunphenon DCF-1 as an EGCG-containing extract. Commercially available banaba tea, green tea, oolong tea or buckwheat tea prepared according to a conventional method was used as tea. The products of the present invention contained the EGCG-containing extract at a concentration of 0.1%(w/v) whereas the control did not. Sensory evaluation was conducted as described in Example 12. The results are shown in Table 19.

**Table 18**

| | Banaba | tea | Green | tea | Oolong | tea | Buckwheat | tea |
|---|---|---|---|---|---|---|---|---|
| | P | C | P | C | P | C | P | C |
| Amount of EGCG added (%, w/v) | 0.1 | 0 | 0.1 | 0 | 0.1 | 0 | 0.1 | 0 |
| P: the product of the present invention; C: the control. | | | | | | | | |

**Table 19:**

| Sensory evaluation | | | | | |
|---|---|---|---|---|---|
| EGCG-containing drink | | Item | | | Total |
| | | Taste | Flavor | Color | |
| Banaba | P | 2.9 | 2.7 | 3.4 | 3.1 |
| tea | C | 3.4 | 3.6 | 3.8 | 3.6 |
| Green | P | 2.7 | 3.1 | 3.2 | 3.0 |
| tea | C | 3.5 | 3.7 | 3.4 | 3.6 |
| Oolong | P | 3.0 | 3.0 | 3.1 | 3.0 |
| tea | C | 3.5 | 3.7 | 3.5 | 3.6 |
| Buckwheat | P | 3.1 | 3.2 | 3.2 | 3.1 |
| tea | C | 3.7 | 3.6 | 3.6 | 3.6 |
| P: the product of the present invention; C: the control. | | | | | |

As shown in Table 19, the drinks of the present invention prepared by combining tea with the EGCG-containing extract were evaluated as novel drinks each having harmonized flavor and taste of tea with good balance, novel amplitude which cannot be provided by tea alone, fulfilled balance of flavor and taste, and bitter taste with stimulant feeling.

The EGCG contents and the caffeine contents of the banaba tea drink, the green tea drink, the oolong tea drink and the buckwheat tea drink of the present invention were 0.05%(w/v) and below detection limit, 0.06%(w/v) and 0.015%(w/v), 0.05%(w/v) and 0.004%(w/v), and 0.05%(w/v) and below detection limit, respectively.

An alcoholic drink prepared by adding ethanol to each of the tea drinks of the present invention at a final concentration of 4%(v/v) exhibited good flavor.

The EGCG content of a commercially available alcoholic drink containing oolong tea was 0.001%(w/v).

(2) Tea drinks each having the composition as shown in Table 20 were prepared. Each drink contained an EGCG-containing extract (Sunphenon BG) at a concentration of 0.05%(w/v) and contained, or did not contain, ethanol at a concentration of 4%(v/v). Specifically, 500 ml of twofold strengthened extract of commercially available black tea was obtained. The components indicated in Table 20 were added to the extract to prepare 1000 ml of lemon tea drink with or without alcohol. The products of the present invention contained the EGCG-containing extract (Sunphenon BG) whereas the control did not. The products were filled into 200-ml cans and sterilized at 83°C for 1 minute to prepare canned products. Sensory evaluation was conducted as described in Example 12. The panel consisted of 10 members. Five grades were used for the sensory evaluation (1: good; 5: bad). The results were expressed using the mean values. The results are shown in Table 21.

**Table 20**

| | With alcohol (%) | Without alcohol (%) |
|---|---|---|
| Tea | 50 | 50 |
| 1/5 lemon juice | 0.1 | 0.1 |
| Citric acid or Na citrate | s.a. | s.a. |
| Vitamin C | 0.02 | 0.02 |
| Sugar (cane) | 4.5 | 4.5 |
| Sunphenon BG | 0.05 | 0.05 |
| Alcohol content (v/v) | 4.0 | 0 |
| pH | 3.7 | 3.7 |

| | | |
|---|---|---|
| s.a.: suitable amount. | | |

**Table 21**

| Lemon tea | Item | | | Total |
|---|---|---|---|---|
| | Taste | Flavor | Color | |
| Lemon tea with alcohol | | | | |
| Product of the invention | 2.8 | 2.9 | 3.2 | 2.9 |
| Control | 3.3 | 3.6 | 3.5 | 3.4 |
| Lemon tea without alcohol | | | | |
| Product of the invention | 2.9 | 3.1 | 3.2 | 3.0 |
| Control | 3.5 | 3.7 | 3.5 | 3.6 |

As shown in Table 21, the lemon tea drinks of the preset invention containing the EGCG-containing extract with or without alcohol were evaluated as novel drinks each having moderately stimulant taste and tightened taste, and having better total balance of taste, flavor and color than the control.

Furthermore, various tea drinks were produced by preparing a green tea drink, an oolong tea drink, a barley tea drink and a black tea drink in which the ratios of EGCG to caffeine were 0.9 or more and the EGCG contents were from 0.01 to 0.2%(w/v), and sterilizing them.

Each tea drink was a green tea drink, an oolong tea drink, a barley tea drink or a black tea drink having a moderately stimulant feeling in which the flavor of EGCG was added to the flavor of the tea extract.

The ratios of EGCG to caffeine of commercially available sterile green tea drinks ranged from 0.7 to 0.8. The EGCG contents were less than 0.01%(w/w).

The ratio of EGCG to caffeine of a commercially available sterile black tea drink was 0.6. The EGCG content was 0.004%(w/w).

### Example 15

Soup was prepared using Sunphenon LA-10 as an EGCG-containing extract. The product of the present invention contained the extract whereas the control did not. The composition of the soup containing the EGCG-containing extract is shown in Table 22. Soup was prepared to have the composition, filled into 200-ml cans, and sterilized by heating at 120°C for 15 minutes to prepare canned products. Sensory evaluation was conducted as described in Example 12. The panel consisted of 10 members. Five grades were used for the sensory evaluation (1: good; 5: bad). The results were expressed using the mean values. The results are shown in Table 23.

**Table 22:**

| Composition of soup containing EGCG-containing extract | | |
|---|---|---|
| | Product of the invention (%, w/v) | Control (%, w/v) |
| EGCG-containing extract | 0.15 | 0 |
| Low strength agar* | 0.25 | 0.25 |
| Whey mineral | 0.60 | 0.60 |
| Pork extract | 0.13 | 0.13 |
| Sugar | 0.10 | 0.10 |
| Pepper | 0.0005 | 0.0005 |
| Vitamin C | 0.02 | 0.02 |
| pH | 5.0 | |
| Brix | 1.1 | |
| Acidity (corresponding citric acid) | 0.025 | |

| | | |
|---|---|---|
| *: from Ina Shokuhin Kogyo | | |
| pH was adjusted using citric acid or sodium citrate. | | |

**Table 23:**

| Sensory evaluation | | | | |
|---|---|---|---|---|
| Soup | Item | | | Total |
| | Taste | Flavor | Color | |
| Product of the invention | 2.6 | 2.8 | 3.1 | 2.8 |
| Control | 3.2 | 3.6 | 3.5 | 3.4 |

As described in Table 23, the product of the present invention was evaluated, as compared with the control, to have amplitude and thick feeling, the flavor and taste of the EGCG-containing extract matched well with pork extract, novel taste which cannot be provided by pork extract alone, and mild flavors in which flavors of vegetable and meat are harmonized each other. The EGCG-containing extract was effective as a hidden flavor in adjusting the flavor and taste. The control tended to lose its balance of flavor and taste. As described above, it was demonstrated that the EGCG-containing extract of the present invention is excellent as a seasoning to be used for cooking based on its properties flavor and taste.

### Example 16

A "furikake" (tastily seasoned dried food for sprinkling on rice) was prepared by mixing 2.4 kg of fish meal, 0.5 kg of salt and 0.3 kg of sodium glutamate (3.2 kg in total) with (the product of the present invention) or without (a control) the addition of a freeze-dried EGCG-containing extract at a ratio of 3 g per kg of the mixture and granulating the resulting mixture according to a conventional method. 0.4 kg of laver and 1.2 kg of sesame were mixed thoroughly with about 3.2 kg of the granulated product. The furikake was sprinkled on boiled rice and sensory evaluation was conducted as described in Example 12. As a result, the product of the present invention was evaluated to have suppressed fish smell derived from fish meal, total amplitude of taste and tightened taste as compare with the control. It was demonstrated to have spice-like functions as a hidden flavor. Thus, the total quality of furikake of the present invention was demonstrated to be improved.

### Example 17

The product of the present invention was prepared by adding 0.02 g of the EGCG-containing extract (Sunphenon 100S) to 100 ml of sake prepared according to a conventional method. Sake without the addition of EGCG was used as a control. Sensory evaluation was conducted as described in Example 12. The results are shown in Table 24.

**Table 24:**

| Sensory evaluation | | |
|---|---|---|
| | The product of the present invention | Control |
| Taste | | |
| Balance | 2.8 | 3.5 |
| Amplitude | 2.7 | 3.6 |
| Aftertaste | 3.0 | 3.6 |
| Flavor | 2.9 | 3.2 |
| Total | 2.8 | 3.4 |

As described in Table 24, the product of the present invention was evaluated to be a luxury product having improved flavor and taste, well balanced taste, tightened taste and excellent amplitude as compared with the control.

### Example 18

The products of the present invention were prepared by adding 0.05 g of the EGCG-containing extract (Sunphenon 100S) to 100 ml of sweet sake or a fermented seasoning prepared according to conventional methods. Products without the addition of EGCG were used as controls. Sensory evaluation was conducted as described in Example 12. The results are shown in Table 25.

**Table 25:**

| Sensory evaluation | | | | |
|---|---|---|---|---|
| | Sweet sake | | Fermented seasoning | |
| | P | C | P | C |
| Balance | 2.9 | 3.0 | 2.9 | 3.2 |
| Flavor | 2.7 | 3.0 | 3.0 | 3.4 |
| Color | 2.7 | 3.0 | 3.1 | 3.5 |
| Total | 2.8 | 3.0 | 2.9 | 3.3 |
| P: the product of the present invention; C: the control. | | | | |

As described in Table 25, the products of the present invention were evaluated to be novel sweet sake and fermented seasoning which have stimulant feeling, totally improved balance of taste, enhanced sweet taste in particular, and improved flavor and taste as compared with the controls.

### Industrial Applicability

As described above in detail, a substance having an antagonistic activity against an estrogen receptor agonist, as well as a food or a drink containing the same can effectively ameliorate or prevent an inappropriate physiological disease state due to an excess amount of estrogen or an endocrine disruptor, i.e., a malignant tumor having estrogen receptor, metrofibroma, endometritis, decrease in sperm number, prostatomegaly or the like.

Furthermore, the present invention provides a food or drink in which a substance belonging to catechins is increased, which has bitter taste with a stimulant feeling, tightened taste, and well balanced taste.

## Claims

1. A pharmaceutical composition for treating or preventing a disease caused by binding of an agonist to estrogen receptor, which contains, as an active ingredient, a substance having an antagonistic activity against an estrogen receptor agonist.

2. The pharmaceutical composition according to claim 1, wherein the substance having an antagonistic activity against an estrogen receptor agonist is at least one compound selected from the group consisting of:
a compound of general formula I: 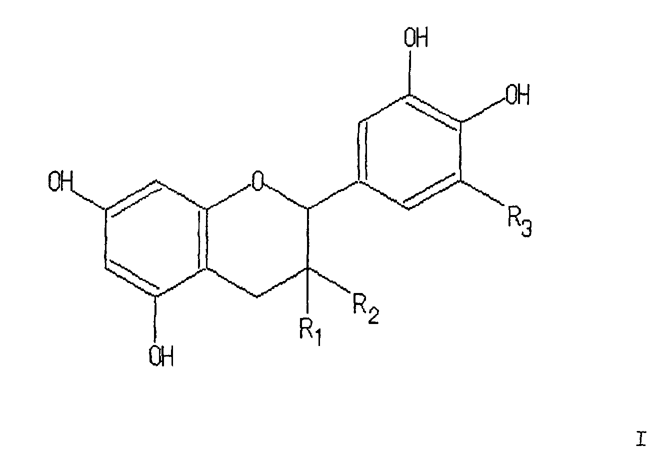
wherein R₁ and R₂ represent -H, -OH or -O-R₄; R₃ represents -H or -OH; R₄ represents a compound of formula II: 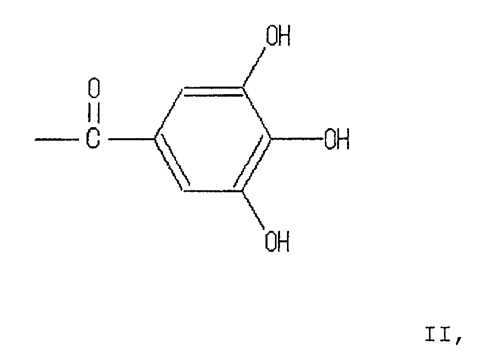 a derivative thereof or a salt thereof;
gallic acid;
a derivative thereof; and
a pharmacologically acceptable salt thereof.

3. The pharmaceutical composition according to claim 1 or 2, wherein the substance having an antagonistic activity against an estrogen receptor agonist is a substance belonging to catechins.

4. The pharmaceutical composition according to claim 3, wherein the substance belonging to catechins is at least one selected from the group consisting of the catechins (a) to (h) below, derivatives thereof, and salts thereof:
(a) epicatechin;
(b) epigallocatechin;
(c) epigallocatechin gallate;
(d) epicatechin gallate;
(e) gallocatechin gallate;
(f) catechin gallate;
(g) catechin; and
(h) gallocatechin.

5. The pharmaceutical composition according to claim 2, wherein the derivative of gallic acid is an ester of gallic acid.

6. The pharmaceutical composition according to claim 5, wherein the ester of gallic acid is an ester of gallic acid with an alcohol having eight or more carbons.

7. The pharmaceutical composition according to claim 5, wherein the ester of gallic acid is octyl gallate, dodecyl gallate or cetyl gallate.

8. The pharmaceutical composition according to any one of claims 1 to 7, wherein the agonist is an endocrine disruptor.

9. The pharmaceutical composition according to claim 8, wherein the endocrine disruptor is at least one selected from compounds classified into the classes (1) to (9) below:
(1) dioxins;
(2) organochlorine compounds;
(3) phenols;
(4) phthalate esters;
(5) aromatic hydrocarbons;
(6) pesticides;
(7) organotin compounds;
(8) estrogens; and
(9) mirex, toxaphene, aldicarb or kepone.

10. The pharmaceutical composition according to claim 9, wherein the endocrine disruptor is at least one selected from the group consisting of zeranol, genistein, coumestrol, o,p-DDT, kepone, methoxychlor, zearaalenone, dioxin, p-octylphenol, nonylphenol, dieldrin, butylbenzylphtalate and bisphenol-A.

11. A drink or food for ameliorating a disease state of or preventing a disease caused by binding of an agonist to estrogen receptor, which contains, which is produced by adding thereto, and/or which is produced by diluting a substance having an antagonistic activity against an estrogen receptor agonist.

12. The drink or food according to claim 11, wherein the substance having an antagonistic activity against an estrogen receptor agonist is at least one compound selected from the group consisting of:
a compound of general formula I: 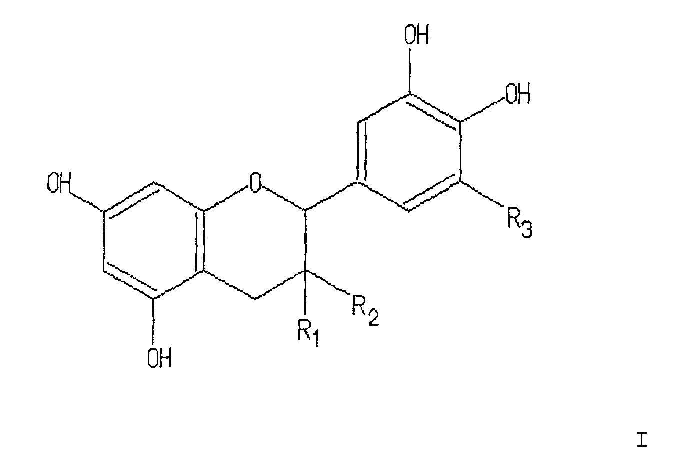
wherein R₁ and R₂ represent -H, -OH or -O-R₄; R₃ represents -H or -OH; R₄ represents a compound of formula II: 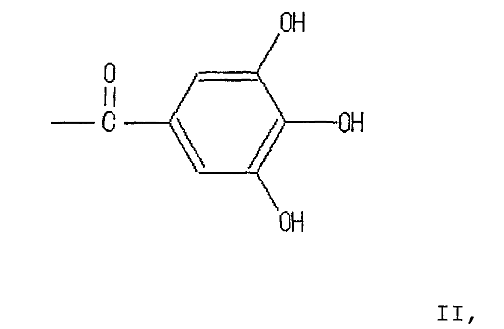 a derivative thereof or a salt thereof;
gallic acid;
a derivative thereof; and
a salt thereof.

13. The drink or food according to claim 11 or 12, wherein the substance having an antagonistic activity against an estrogen receptor agonist is a substance belonging to catechins.

14. The drink or food according to claim 13, wherein the substance belonging to catechins is at least one selected from the group consisting of the catechins (a) to (h) below, derivatives thereof, and salts thereof:
(a) epicatechin;
(b) epigallocatechin;
(c) epigallocatechin gallate;
(d) epicatechin gallate;
(e) gallocatechin gallate;
(f) catechin gallate;
(g) catechin; and
(h) gallocatechin.

15. The drink or food according to claim 12, wherein the derivative of gallic acid is an ester of gallic acid.

16. The drink or food according to claim 15, wherein the ester of gallic acid is an ester of gallic acid with an alcohol having eight or more carbons.

17. The drink or food according to claim 16, wherein the ester of gallic acid is octyl gallate, dodecyl gallate or cetyl gallate.

18. The drink or food according to any one of claims 11 to 16, wherein the agonist is an endocrine disruptor.

19. The drink or food according to claim 18, wherein the endocrine disruptor is at least one selected from compounds classified into the classes (1) to (9) below:
(1) dioxins;
(2) organochlorine compounds;
(3) phenols;
(4) phthalate esters;
(5) aromatic hydrocarbons;
(6) pesticides;
(7) organotin compounds;
(8) estrogens; and
(9) mirex, toxaphene, aldicarb or kepone.

20. The drink or food according to claim 19, wherein the endocrine disruptor is at least one selected from the group consisting of zeranol, genistein, coumestrol, o,p-DDT, kepone, methoxychlor, zearaalenone, dioxin, p-octylphenol, nonylphenol, dieldrin, butylbenzylphtalate and bisphenol-A.

21. An alcoholic drink containing a substance belonging to catechins, which is produced by adding thereto a substance belonging to catechins.

22. The alcoholic drink according to claim 21, wherein the substance belonging to catechins is epigallocatechin gallate.

23. The alcoholic drink according to claim 21, which contains a tea extract.

24. The alcoholic drink according to claim 22 or 23, which contains caffeine at a ratio of epigallocatechin gallate to caffeine of 0.9 or more.

25. The alcoholic drink according to any one of claims 21 to 24, which contains epigallocatechin gallate at a concentration of 0.002 to 0. 75%(w/w).

26. The alcoholic drink according to any one of claims 21 to 25, which has a thick feeling of tea as well as good amplitude and balance of the taste.

27. A tea drink, which is produced by a method comprising a step of sterilization, which contains epigallocatechin gallate at a concentration of 0.01 to 0.5%(w/w), wherein the ratio of epigallocatechin gallate to caffeine is 0.9 or more.
